# EUROPEAN PATENT SPECIFICATION

(11) **EP 4 248 880 B1**
(45) Date of publication and mention of the grant of the patent: **01.01.2025**
(21) Application number: 21894399.1
(22) Date of filing: 18.10.2021
(51) Int. Cl.: A61B 8/08, A61B 8/14, A61B 8/00, G06T 7/136, G06T 7/11

(54) **IMAGE PROCESSING DEVICE AND METHOD FOR CONTROLLING IMAGE PROCESSING DEVICE**
BILDVERARBEITUNGSVORRICHTUNG UND VERFAHREN ZUR STEUERUNG DER BILDVERARBEITUNGSVORRICHTUNG
DISPOSITIF DE TRAITEMENT D'IMAGE ET PROCÉDÉ DE COMMANDE D'UN DISPOSITIF DE TRAITEMENT D'IMAGE

(30) Priority: 19.11.2020 JP 2020192655
(43) Date of publication of application: 27.09.2023
(73) Proprietor: FUJIFILM Corporation, Tokyo 106-8620 (JP)
(72) Inventor: EBATA, Tetsurou, Tokyo 106-8620 (JP)
(74) Representative: Dehns Germany Partnerschaft mbB
(86) International application number: PCT/JP2021/038407
(87) International publication number: WO 2022/107525

(56) References cited:
- WO-A1-2020/044769
- WO-A1-2020/087732
- JP-A- 2013 180 160
- JP-A- 2016 159 116
- JP-A- 2017 221 774
- US-A1- 2021 137 492

## Description

### BACKGROUND OF THE INVENTION

### 1. Field of the Invention

The present invention relates to an image processing apparatus that detects a blood vessel in an ultrasound image and a control method of the image processing apparatus.

### 2. Description of the Related Art

In the related art, in preparation for inserting a puncture needle into a blood vessel of a subject, an ultrasound image of the subject is observed to confirm the blood vessel in the ultrasound image. It is generally known that a certain degree of skill is required for an examiner such as a doctor to observe the ultrasound image and accurately recognize a position of the blood vessel. Therefore, in order that the examiner confirms the blood vessel in the ultrasound image easily, for example, as disclosed in WO2012/147505A, an apparatus that detects a blood vessel in an ultrasound image by analyzing the ultrasound image has been developed. WO 2020/044769 discloses an ultrasound diagnostic apparatus which discriminates whether a detected blood vessel is a vein or an artery. WO 2020/087732 relates to a neural network-based method and system for vein and artery identification.

### SUMMARY OF THE INVENTION

Here, for example, in a case of a subject having a high body fat, the ultrasound image has high brightness on the whole, and an artifact may appear in the blood vessel. In addition, for example, in a case in which gas is accumulated in the subject, a boundary between the blood vessel and surrounding tissue in the ultrasound image may be blurred. As described above, it is known that the appearance of blood vessels in the ultrasound image differs depending on the subject. Therefore, even though the technique disclosed in WO2012/147505A is used, the blood vessel in the ultrasound image may not be accurately detected depending on the subject.

An object of the present invention is to provide an image processing apparatus that can detect a blood vessel in an ultrasound image with high accuracy, and a control method of the image processing apparatus.

The present invention provides an image processing apparatus as claimed in claim 1.

The image processing apparatus can comprise an ultrasound probe and an image generation unit configured to generate the ultrasound image analyzed by the certainty calculation unit based on transmission and reception of an ultrasound beam using the ultrasound probe.

In addition, the image processing apparatus can comprise a certainty memory configured to store the certainty calculated by the certainty calculation unit.

Furthermore, the image processing apparatus can comprise an apparatus control unit configured to control storage of the certainty in the certainty memory.

The apparatus control unit can determine that the ultrasound probe is stationary, and store the certainty in the certainty memory in a case in which it is determined that the ultrasound probe is stationary for a determined time or longer.

In addition, the apparatus control unit can determine that the ultrasound probe is in contact with a body surface of the subject, and in a case in which it is determined that the ultrasound probe is in contact with the body surface of the subject, store the certainty in the certainty memory.

In addition, the apparatus control unit can store the certainty in the certainty memory in a case in which it is determined that a moving speed of the ultrasound probe is lower than a determined moving speed.

In addition, the apparatus control unit can store the certainty in the certainty memory, the certainty being calculated based on the ultrasound image having a frame selected by a frame interval according to a moving speed of the ultrasound probe in the ultrasound image having a plurality of frames generated by the image generation unit.

The certainty threshold value change unit can calculate a change value by multiplying a maximum value of the plurality of certainties calculated for the ultrasound image having a plurality of frames by a determined ratio, and change the certainty threshold value to the change value.

In addition, the certainty threshold value change unit can calculate a change value by statistically analyzing the plurality of certainties calculated for the ultrasound image having a plurality of frames, and change the certainty threshold value to the change value.

It is preferable that the certainty threshold value change unit changes the certainty threshold value to the change value in a case in which the change value is lower than the certainty threshold value of the blood vessel detection unit.

The image processing apparatus can comprise a change notification unit configured to notify a user of a change of the certainty threshold value.

In addition, the image processing apparatus can comprise a certainty threshold value memory configured to store the certainty threshold value changed by the certainty threshold value change unit for each subject.

In addition, the image processing apparatus can include an input device for a user to perform an input operation and a manual change unit configured to change the certainty threshold value based on the input operation via the input device.

The present invention also provides a control method of an image processing apparatus as claimed in claim 15.

According to the present invention, the image processing apparatus comprises a certainty calculation unit configured to analyze an ultrasound image having a plurality of frames of a subject for each frame and calculate a certainty of each blood vessel-like structure in the ultrasound image, a blood vessel detection unit configured to detect the blood vessel-like structure in which the certainty calculated by the certainty calculation unit is higher than a certainty threshold value; and a certainty threshold value change unit configured to change the certainty threshold value based on a plurality of the certainties calculated by the certainty calculation unit, so that it is possible to detect the blood vessel in the ultrasound image with high accuracy.

### BRIEF DESCRIPTION OF THE DRAWINGS

Fig. 1 is a block diagram illustrating a configuration of an image processing apparatus according to a first embodiment of the present invention.
Fig. 2 is a diagram illustrating an example of an ultrasound image including a blood vessel.
Fig. 3 is a diagram illustrating an example of a seek bar displayed on a monitor in the first embodiment of the present invention.
Fig. 4 is a diagram illustrating an example of a dialog panel in the first embodiment of the present invention.
Fig. 5 is a diagram illustrating another example of a dialog panel in the first embodiment of the present invention.
Fig. 6 is a flowchart illustrating an operation of the image processing apparatus in the first embodiment of the present invention.
Fig. 7 is a block diagram illustrating a configuration of an ultrasound diagnostic apparatus according to a second embodiment of the present invention.
Fig. 8 is a block diagram illustrating a configuration of a transmission and reception circuit in the second embodiment of the present invention.
Fig. 9 is a block diagram illustrating a configuration of an image generation unit in second embodiment of the present invention.
Fig. 10 is a flowchart illustrating an operation of the ultrasound diagnostic apparatus according to the second embodiment of the present invention.

### DESCRIPTION OF THE PREFERRED EMBODIMENTS

Hereinafter, embodiments of the present invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In the present specification, the terms "same" and "identical" include an error range generally allowed in the technical field.

### First Embodiment

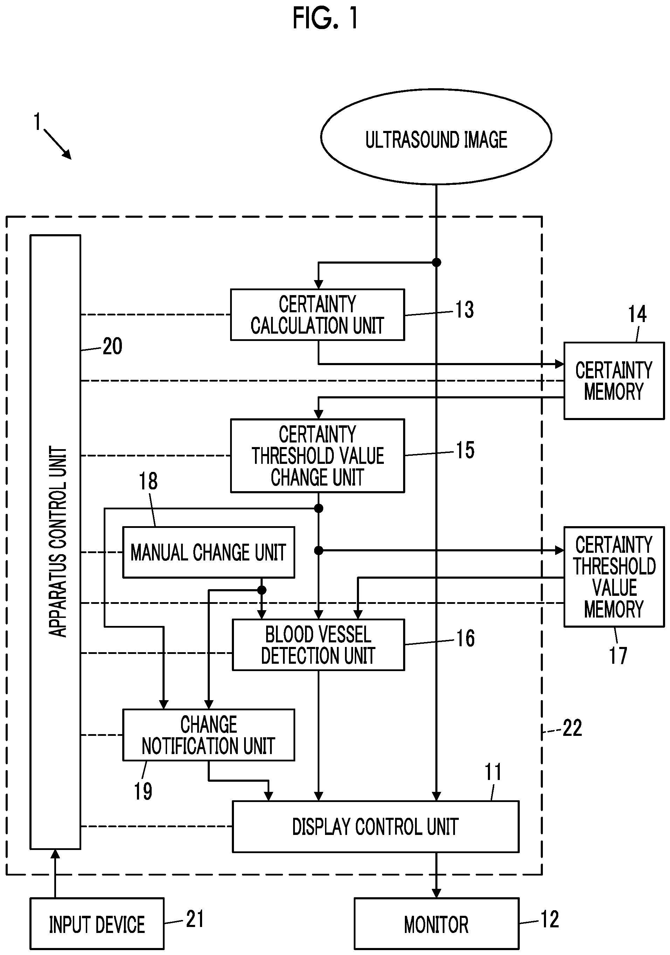

Fig. 1 illustrates a configuration of an image processing apparatus 1 according to a first embodiment of the present invention. The image processing apparatus 1 receives an ultrasound image from external equipment (not illustrated), such as an ultrasound diagnostic apparatus, and displays and analyzes the ultrasound image.

The image processing apparatus 1 comprises a display control unit 11, and a monitor 12 is connected to the display control unit 11. In addition, the image processing apparatus 1 comprises a certainty calculation unit 13, a certainty memory 14 is connected to a certainty calculation unit 13, and a certainty threshold value change unit 15 is connected to the certainty memory 14. In addition, a blood vessel detection unit 16, a certainty threshold value memory 17, and a change notification unit 19 are connected to the certainty threshold value change unit 15. The certainty threshold value memory 17 is connected to the blood vessel detection unit 16. In addition, the image processing apparatus 1 comprises a manual change unit 18, and the manual change unit 18 is connected to the blood vessel detection unit 16 and the change notification unit 19. In addition, the blood vessel detection unit 16 and the change notification unit 19 are connected to the display control unit 11.

In addition, an apparatus control unit 20 is connected to the display control unit 11, the certainty calculation unit 13, the certainty memory 14, the certainty threshold value change unit 15, the blood vessel detection unit 16, the certainty threshold value memory 17, the manual change unit 18, and the change notification unit 19. In addition, an input device 21 is connected to the apparatus control unit 20.

In addition, the display control unit 11, the certainty calculation unit 13, the certainty threshold value change unit 15, the blood vessel detection unit 16, the manual change unit 18, the change notification unit 19, and the apparatus control unit 20 constitute a processor 22.

In addition, an ultrasound image is input to the display control unit 11 and the certainty calculation unit 13 from external equipment (not illustrated) such as a so-called ultrasound diagnostic apparatus.

The certainty calculation unit 13 analyzes the ultrasound image of a subject for each frame and calculates certainty of a blood vessel in the ultrasound image. Here, the certainty of the blood vessel in the ultrasound image is an index representing likelihood of a blood vessel-like structure included in the ultrasound image, and is represented by, for example, a probability that the structure is a blood vessel. For each ultrasound image, the certainty calculation unit 13 calculates the certainty for each blood vessel-like structure in the ultrasound image. Here, the blood vessels for which the certainty is calculated include veins and arteries.

The certainty calculation unit 13 can calculate the certainty of the blood vessel in the ultrasound image, for example, by applying a method using simple template matching, a machine learning technique disclosed in Csurka et al.: Visual Categorization with Bags of Keypoints, Proc. of ECCV Workshop on Statistical Learning in Computer Vision, pp. 59 to 74 (2004), or a general image recognition technique using deep learning disclosed in Krizhevsk et al.: ImageNet Classification with Deep Convolutional Neural Networks, Advances in Neural Information Processing Systems 25, pp. 1106 to 1114 (2012).

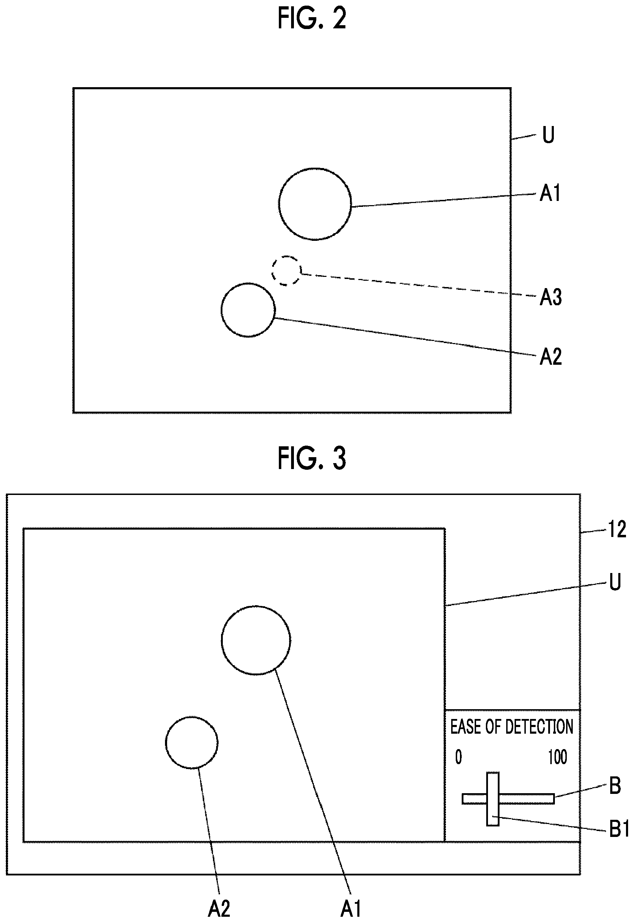

Fig. 2 illustrates an example of an ultrasound image U including blood vessel-like structures A1, A2, and A3. The certainty calculation unit 13 calculates the certainty for each of the structures A1, A2, and A3 in a case of calculating the certainty for the ultrasound image U.

The certainty memory 14 is a memory for storing the certainty calculated by the certainty calculation unit 13. The certainty stored in the certainty memory 14 is transmitted to the certainty threshold value change unit 15 under control of the apparatus control unit 20.

In addition, as the certainty memory 14, for example, recording media such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory) can be used.

The blood vessel detection unit 16 has a certainty threshold value with respect to the certainty of the blood vessel, and detects a blood vessel in which the certainty calculated by the certainty calculation unit 13 is higher than the certainty threshold value. In a case in which the certainty calculated by the certainty calculation unit 13 is equal to or lower than the certainty threshold value, the blood vessel detection unit 16 does not detect a blood vessel having the certainty. For example, among the three structures A1, A2, and A3 illustrated in Fig. 2, in a case in which the certainties for the structures A1 and A2 are larger than the certainty threshold value and the certainty for the structure A3 is equal to or lower than the certainty threshold value, structures A1 and A2 are detected as blood vessels, and structure A3 is not detected as blood vessels.

In this way, the ease of detecting the blood vessel is determined by the certainty threshold value. That is, the higher the certainty threshold value is set, the more difficult it is for the structures A1, A2, and A3 to be detected as blood vessels, and the lower the certainty threshold value is set, the easier it is for the structures A1, A2, and A3 to be detected as blood vessels.

The certainty threshold value change unit 15 calculates a certainty change value for each of the structures A1, A2, and A3 and changes the certainty threshold value of the blood vessel detection unit 16 to a change value based on a plurality of certainties calculated by the certainty calculation unit 13 for the ultrasound image U having a plurality of frames.

For example, the certainty threshold value change unit 15 can calculate a change value by multiplying the maximum value of the plurality of certainties calculated for the ultrasound image U having a plurality of frames by a determined ratio that is smaller than 1.0, such as 0.8.

At this time, it is desirable that the certainty threshold value change unit 15 excludes the certainty having a value corresponding to a so-called outlier. The certainty threshold value change unit 15 can calculate, for example, an average value and a standard deviation of the plurality of certainties, and further, can exclude the certainty corresponding to the outlier by excluding the certainty having a value equal to or larger than a sum of three times the calculated standard deviation and the average value from the plurality of certainties.

In addition, the certainty threshold value change unit 15 can also calculate the change value by statistically analyzing the plurality of certainties calculated for the ultrasound image U having a plurality of frames. Statistical analysis of the plurality of certainties refers to analysis of the distribution of values of the plurality of certainties. The certainty threshold value change unit 15 can, for example, sort the plurality of certainties in ascending order or in descending order, and calculate, as a change value, a value of the certainty located in a rank corresponding to a determined ratio such as the top 20% to the total number of the plurality of certainties, with the highest certainty as first place. In addition, the certainty threshold value change unit 15 also can, for example, sort the plurality of certainties in ascending order or in descending order, and calculate, as a change value, the certainty located in a determined rank relative to the total number of the plurality of certainties, with the highest certainty as first place.

In addition, as an example of statistically analyzing the plurality of certainties, the certainty threshold value change unit 15 can also calculate, for example, as a change value, a sum of a constant multiple of a standard deviation of the plurality of certainties and an average value of the plurality of certainties.

The certainty threshold value memory 17 is a memory that stores the certainty threshold value changed by the certainty threshold value change unit 15 separately for each subject. The certainty threshold value stored in the certainty threshold value memory 17 is, for example, transmitted to the blood vessel detection unit 16 at a start of an examination of a subject corresponding to the certainty threshold value under the control of the apparatus control unit 20, and used as an initial value of the certainty threshold value of the blood vessel detection unit 16.

As the certainty threshold value memory 17, for example, recording media such as a flash memory, an HDD, an SSD, an FD, an MO disk, an MT, a RAM, a CD, a DVD, an SD card, or a USB memory can be used.

The input device 21 is used for the user to perform an input operation. For example, the input device 21 is composed of a device such as a keyboard, a mouse, a trackball, a touchpad, and a touch panel for the user to perform the input operation.

The manual change unit 18 changes the certainty threshold value to a value designated based on the input operation of the user via the input device 21. Examples of the input operation of the user via the input device 21 include, for example, an input operation using a seek bar B illustrated in Fig. 3. The seek bar B has a slide button B1 that slides between both end portions thereof, and it is possible to designate a certainty threshold value corresponding to a position of the slide button B1. For example, in order to make it easy for the user to intuitively grasp the meaning of the certainty threshold value, the monitor 12 can display a left end of the seek bar B as "ease of detection 0" and a right end of the seek bar B as "ease of detection 100" by making a determined range for the certainty correspond to a range of the ease of detection of 0 to 100.

In this case, the user can designate a lower certainty threshold value as the slide button B1 is closer to the "ease of detection 0" at the left end of the seek bar B, and can designate a higher certainty threshold value as the slide button B1 is closer to the "ease of detection 100" at the right end of the seek bar B.

The change notification unit 19 notifies the user of the change of the certainty threshold value.

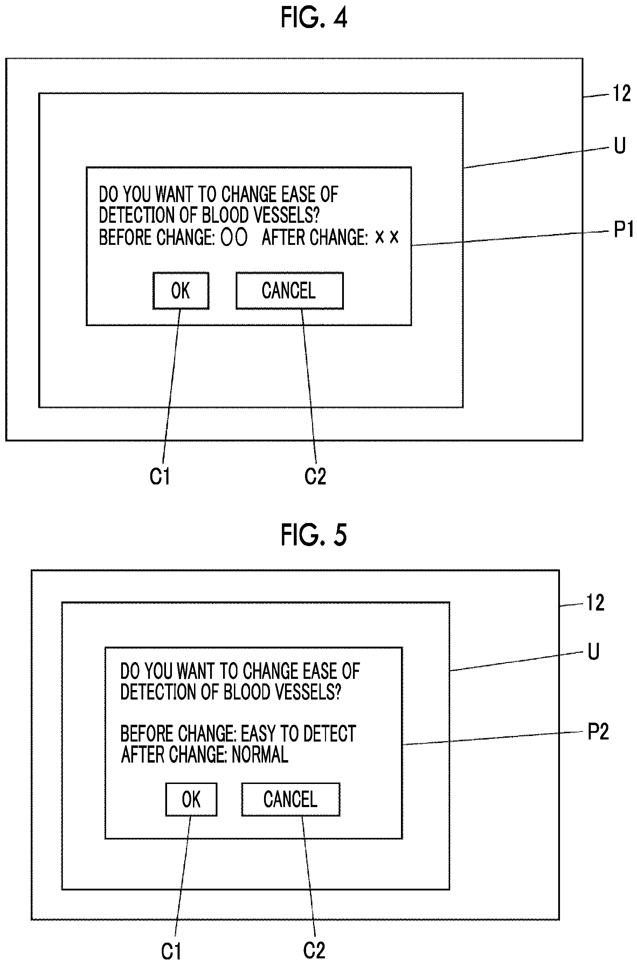

As illustrated in Fig. 4, for example, the change notification unit 19 can cause the monitor 12 to display a dialog panel P1 for selecting whether to execute or cancel the change of the certainty threshold value in a case in which the certainty threshold value is about to be changed. In this dialog panel P1, a message "Do you want to change ease of detection of blood vessels?", values of the ease of detection before and after the change, an execution button C1 for executing the change of the certainty threshold value, and a cancel button C2 for canceling the change of the certainty threshold value are included.

In addition, in a case in which the certainty threshold value is about to be changed, the change notification unit 19 can classify, for example, the ease of detection of the blood vessels into a plurality of stages such as "very difficult to detect", "difficult to detect", "normal", "easy to detect", and "very easy to detect", and can display the classified stages instead of the values of the ease of detection before and after the change in the dialog panel P1. For example, in a dialog panel P2 illustrated in Fig. 5, "easy to detect" is displayed as the ease of detection before the change, and "normal" is displayed as the ease of detection after the change.

The apparatus control unit 20 controls each unit of the image processing apparatus 1 according to a program or the like recorded in advance.

The display control unit 11 performs, under the control of the apparatus control unit 20, predetermined processing on the ultrasound image U and the structures A1, A2, and the like, which are detected as blood vessels by the blood vessel detection unit 16 and displays them on the monitor 12.

The monitor 12 performs various types of display under control of the display control unit 11. For example, the monitor 12 includes a display device such as a liquid crystal display (LCD) or an organic electroluminescence display (organic EL display).

The processor 22 having the display control unit 11, the certainty calculation unit 13, the certainty threshold value change unit 15, the blood vessel detection unit 16, the manual change unit 18, the change notification unit 19, and the apparatus control unit 20 is configured of a central processing unit (CPU) and a control program causing the CPU to execute various types of processing. However, the processor 22 may be configured using a field programmable gate array (FPGA), a digital signal processor (DSP), an application specific integrated circuit (ASIC), a graphics processing unit (GPU), or other integrated circuits (ICs) or may be configured by combining thereof.

In addition, the display control unit 11, the certainty calculation unit 13, the certainty threshold value change unit 15, the blood vessel detection unit 16, the manual change unit 18, the change notification unit 19, and the apparatus control unit 20 of the processor 22 can be partially or wholly integrated into one CPU or the like.

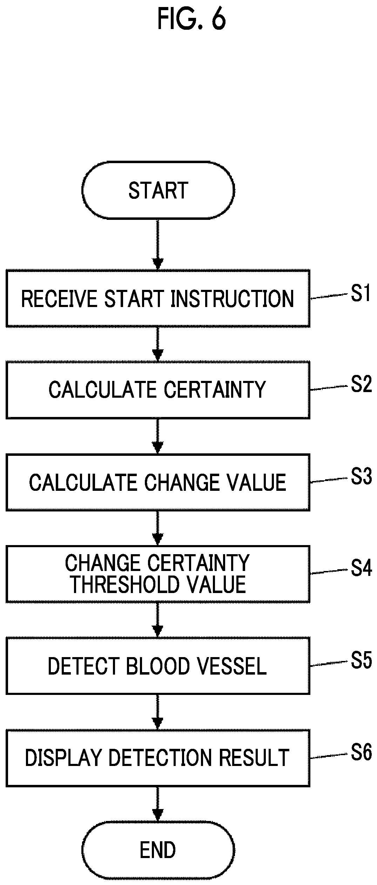

Next, an operation of the image processing apparatus 1 according to the first embodiment of the present invention will be described using the flowchart illustrated in Fig. 6. It is assumed that the ultrasound image U having a plurality of frames, in which the same part of the same subject is imaged, is previously input to the image processing apparatus 1 from external equipment such as an ultrasound diagnostic apparatus (not illustrated).

First, in step S1, the apparatus control unit 20 receives an instruction to start a series of processing for detecting the blood vessel (processing of step S2 to step S6). For example, in a case in which an instruction to start a series of processing for detecting the blood vessel is given by a user's input operation via the input device 21, the instruction is received by the apparatus control unit 20.

Next, in step S2, the certainty calculation unit 13 calculates the certainty of the blood vessel for each ultrasound image U input from the external equipment such as an ultrasound diagnostic apparatus. For example, as illustrated in Fig. 2, in a case in which three blood vessel-like structures A1, A2, and A3 are included in the ultrasound image U, the certainty is calculated for each of the three structures A1, A2, and A3 in each ultrasound image U.

The plurality of certainties calculated in this way are stored in the certainty memory 14.

In subsequent step S3, the certainty threshold value change unit 15 calculates a change value for the certainty threshold value of the blood vessel detection unit 16 for each of the structures A1 to A3 in the ultrasound image U based on the plurality of certainties calculated in step S2 and stored in the certainty memory 14. For example, the certainty threshold value change unit 15 calculates a change value by multiplying the maximum value of the plurality of certainties calculated for the ultrasound image U having a plurality of frames by a determined ratio such as 0.8.

Here, for example, an ultrasound image of a subject having a high body fat generally has high brightness on the whole, an artifact may be included in the blood vessel in the ultrasound image, and in a case in which gas is accumulated in the subject, a boundary between the blood vessel in the ultrasound image and the surrounding tissue may be blurred, and it is known that the blood vessel in the ultrasound image becomes difficult to see depending on the subject. In this way, in a case in which the certainty of the blood vessel is calculated for the ultrasound image in which the blood vessel is difficult to see, a certainty having a value lower than a certainty calculated for the ultrasound image in which the blood vessel appears normal is calculated.

Since the change value calculated by the certainty threshold value change unit 15 has a value lower than the maximum value of the plurality of certainties calculated for the ultrasound image U having a plurality of frames of the same subject, even in a case in which the structures A1 to A3 in the ultrasound image U are difficult to see because of a state of the subject, the structures A1 to A3 can be easily detected as blood vessels.

In step S4, the certainty threshold value change unit 15 changes the certainty threshold value of the blood vessel detection unit 16 to the change value calculated in step S3.

Here, in a case in which the certainty threshold value change unit 15 attempts to change the certainty threshold value of the blood vessel detection unit 16, the change notification unit 19 can notify the user of the change of the certainty threshold value. As illustrated in Fig. 4, for example, the change notification unit 19 can cause the monitor 12 to display the dialog panel P1 for allowing the user to select whether to execute or cancel the change of the certainty threshold value. In this case, in a case in which the user selects the execution button C1 via the input device 21, the certainty threshold value change unit 15 changes the certainty threshold value of the blood vessel detection unit 16 to the change value calculated in step S3. In addition, in a case in which the user selects the cancel button C2, the change of the certainty threshold value by the certainty threshold value change unit 15 is canceled.

In this way, by notifying the user of the change of the certainty threshold value, the user can clearly grasp that the ease of detection of the blood vessel has been changed.

In addition, in step S4, in addition to the change of the certainty threshold value by the certainty threshold value change unit 15, the certainty threshold value can also be changed by the manual change unit 18. In this case, for example, as illustrated in Fig. 3, the user slides the slide button B 1 of the seek bar B via the input device 21 to designate the certainty threshold value, and the manual change unit 18 changes the certainty threshold value to a value designated by the user.

In this way, by changing the certainty threshold value based on the input operation via the input device 21, the ease of detection of the blood vessel can be adjusted in more detail.

In addition, the certainty threshold value changed in step S4 is stored in the certainty threshold value memory 17 for each subject. Here, the stored certainty threshold value is, for example, read out under the control of the apparatus control unit 20 at the start of the next examination for the same subject, and can be used as an initial value of the certainty threshold value of the blood vessel detection unit 16.

In step S5, the blood vessel detection unit 16 detects a blood vessel having a certainty higher than the certainty threshold value changed in step S4. For example, in a case in which the certainties calculated for the structures A1 and A2 illustrated in Fig. 2 are higher than the certainty threshold value, and the certainty calculated for the structure A3 is equal to or lower than the certainty threshold value, the blood vessel detection unit 16 detects the structures A1 and A2 as blood vessels, and does not detect the structure A3 as blood vessels among the structures A1 to A3.

Finally, in step S6, the blood vessel detection unit 16 highlights the structures A1 and A2 detected as blood vessels in step S5 and displays the structures A1 and A2 on the monitor 12. Although not illustrated, the blood vessel detection unit 16 can superimpose the contour lines of the structures A1 and A2 detected as blood vessels on the ultrasound image U and display them on the monitor 12, for example. In addition, for example, the structures A1 and A2 can be highlighted by giving them different colors from their surroundings. In this manner, the user can easily grasp the structures A1 and A2 as blood vessels.

From the above, with the image processing apparatus 1 according to the first embodiment of the present invention, since the certainty threshold value is changed based on the plurality of certainties calculated by the certainty calculation unit 13 for the ultrasound image U having a plurality of frames, even in a case in which the blood vessels in the ultrasound image U are difficult to see because of the state of the subject, the blood vessels can be detected with high accuracy.

In step S4, it is desirable that the certainty threshold value change unit 15 does not execute the change of the certainty threshold value in order to prevent the detection of the blood vessel from becoming difficult in a case in which the change value calculated in step S3 has a value equal to or larger than the initial value of the certainty threshold value of the blood vessel detection unit 16.

In addition, in a case in which the certainty threshold value is very low compared to a general value, the risk of falsely detecting the blood vessel increases, and conversely, in a case in which the certainty threshold value is very high, there is a risk that the blood vessel cannot be detected. Therefore, it is desirable that an upper limit value and a lower limit value are set for the certainty threshold value. For example, 0.8 times the initial value of the certainty threshold value can be set as the lower limit value, and 1.2 times the certainty threshold value can be set as the upper limit value. As a more specific example, assuming that the initial value of the certainty threshold value is 0.75, the lower limit value can be 0.60 and the upper limit value can be 0.90.

In addition, although it is described that the change notification unit 19 notifies the user of the change of the certainty threshold value at a timing at which the certainty threshold value change unit 15 attempts to change the certainty threshold value, it is also possible to notify the user that the certainty threshold value has already been changed. Accordingly, the user can smoothly confirm the blood vessel while clearly grasping that the certainty threshold value has been changed.

### Second Embodiment

The image processing apparatus 1 according to the first embodiment receives the ultrasound image U from the external equipment (not illustrated), but the present invention is not particularly limited to this aspect. For example, the image processing apparatus 1 may be an ultrasound diagnostic apparatus comprising an ultrasound probe, or the ultrasound image U captured by the ultrasound probe can also be analyzed.

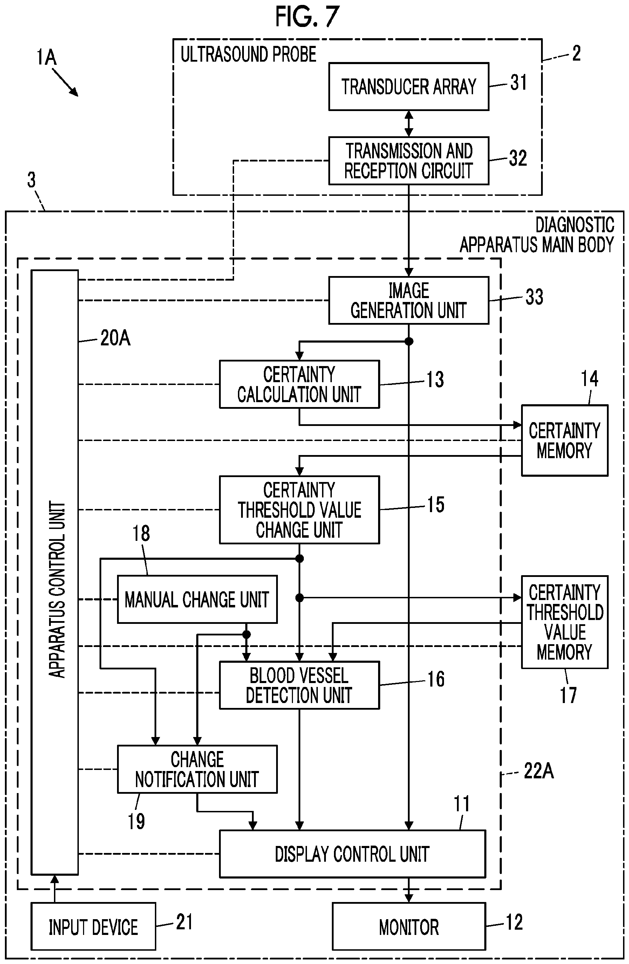

Fig. 7 illustrates a configuration of an ultrasound diagnostic apparatus 1A according to a second embodiment.

The ultrasound diagnostic apparatus 1A comprises an ultrasound probe 2 and a diagnostic apparatus main body 3, and the ultrasound probe 2 and the diagnostic apparatus main body 3 are connected to each other.

The ultrasound probe 2 comprises a transducer array 31. A transmission and reception circuit 32 is connected to the transducer array 31.

In the diagnostic apparatus main body 3, an image generation unit 33 is added in the image processing apparatus 1 of the first embodiment, an apparatus control unit 20A is comprised instead of the apparatus control unit 20, and a processor 22A including the image generation unit 33 is configured instead of the processor 22. The image generation unit 33 is connected to the transmission and reception circuit 32. In addition, the display control unit 11 and the certainty calculation unit 13 are connected to the image generation unit 33. The apparatus control unit 20A is further connected to the transmission and reception circuit 32 and the image generation unit 33 as compared with the apparatus control unit 20 according to the first embodiment.

The transducer array 31 of the ultrasound probe 2 has a plurality of transducers arranged in a one-dimensional or two-dimensional manner. Each transducer transmits an ultrasound wave in response to a drive signal supplied from the transmission and reception circuit 32, receives an ultrasound echo from a subject, and outputs a signal based on the ultrasound echo. For example, each transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

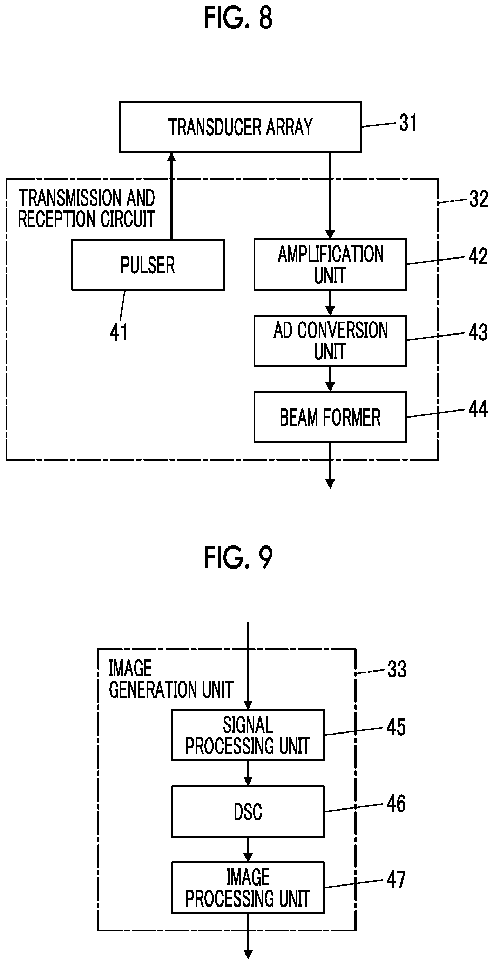

The transmission and reception circuit 32 transmits the ultrasound wave from the transducer array 31 and generates a sound ray signal based on a received signal acquired by the transducer array 31 under control of the apparatus control unit 20A. As illustrated in Fig. 8, the transmission and reception circuit 32 has a pulser 41 connected to the transducer array 31, an amplification unit 42, an analog digital (AD) conversion unit 43, and a beam former 44 that are sequentially connected in series from the transducer array 31.

The pulser 41 includes, for example, a plurality of pulse generators and supplies each driving signal to the plurality of transducers while adjusting the amount of delay such that the ultrasound waves transmitted from the plurality of transducers of the transducer array 31 form an ultrasound beam, based on a transmission delay pattern selected in response to a control signal from the apparatus control unit 20A. In this way, in a case in which a pulsed or continuous-wave voltage is applied to the electrodes of the transducers of the transducer array 31, the piezoelectric body expands and contracts to generate a pulsed or continuous-wave ultrasound wave from each of the transducers. An ultrasound beam is formed from a combined wave of the ultrasound waves.

The transmitted ultrasound beam is reflected by, for example, a target, such as a part of the subject, and propagates toward the transducer array 31 of the ultrasound probe 2. In this way, the ultrasound echo propagating toward the transducer array 31 is received by each transducer constituting the transducer array 31. In this case, each transducer constituting the transducer array 31 receives propagated ultrasound echo, expands and contracts to generate a received signal which is an electric signal, and outputs the received signal to the amplification unit 42.

The amplification unit 42 amplifies the signal input from each transducer constituting the transducer array 31 and transmits the amplified signal to the AD conversion unit 43. The AD conversion unit 43 converts the signal transmitted from the amplification unit 42 into digital reception data and transmits the reception data to the beam former 44. The beam former 44 performs so-called reception focus processing by giving a delay to each piece of reception data converted by the AD conversion unit 43 conforming to acoustic velocity or distribution of acoustic velocity set based on a reception delay pattern selected in response to a control signal from the apparatus control unit 20A and performing addition. With the reception focus processing, each piece of reception data converted in the AD conversion unit 43 is subjected to phasing addition, and a sound ray signal in which a focus of the ultrasound echo is narrowed is acquired. This sound ray signal is transmitted to the image generation unit 33.

As illustrated in Fig. 9, the image generation unit 33 has a configuration in which a signal processing unit 45, a digital scan converter (DSC) 46, and an image processing unit 47 are sequentially connected in series.

The signal processing unit 45 performs correction of attenuation of the sound ray signal transmitted from the transmission and reception circuit 32 due to a distance depending on a depth of a reflection position of an ultrasound wave, and then, executes envelope detection processing, thereby generating a B mode image signal that is tomographic image information related to a tissue in the subject.

The DSC 46 converts (raster-converts) the B mode image signal generated in the signal processing unit 45 into an image signal conforming to a normal television signal scanning system.

The image processing unit 47 performs various types of necessary image processing such as gradation processing on the B mode image signal input from the DSC 46, and then transmits the B mode image signal in response to a command from the apparatus control unit 20 to the display control unit 11 and the certainty calculation unit 13. The B mode image signal subjected to the image processing by the image processing unit 47 is simply referred to as an ultrasound image U.

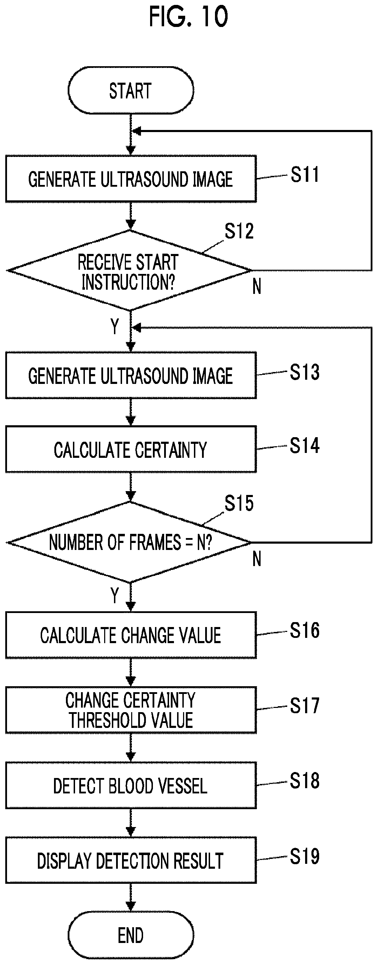

Next, an operation of the ultrasound diagnostic apparatus 1A according to the second embodiment will be described with reference to a flowchart illustrated in Fig. 10. Steps S14 and S16 to S19 in this flowchart are the same as steps S2 and S3 to S6 in the flowchart according to the first embodiment illustrated in Fig. 6, respectively.

First, in step S11, capturing of the ultrasound image U is performed in a state where the ultrasound probe 2 is in contact with the body surface of the subject by the user such that the blood vessel of the subject to be examined is imaged.

At this point, the transmission and reception circuit 32, under the control of the apparatus control unit 20A, generates the sound ray signal by performing the reception focus processing using the sound speed value set in advance. The sound ray signal generated by the transmission and reception circuit 32 in such a manner is transmitted to the image generation unit 33. The image generation unit 33 generates the ultrasound image U using the sound ray signal transmitted from the transmission and reception circuit 32. The ultrasound image U generated in such a manner is transmitted to the display control unit 11 and is displayed on the monitor 12.

In step S12, the apparatus control unit 20A determines whether or not an instruction to start a series of processing for detecting the blood vessel (processing of step S13 to step S19) is received. In a case in which the instruction to start a series of processing for detecting the blood vessel is not input by an input operation of the user via the input device 21, the apparatus control unit 20A determines that, for example, this instruction is not received and returns to step S11. In a case in which the ultrasound image U is newly generated in step S11, the processing proceeds to step S12. In this way, step S11 and step S12 are repeated while it is determined that the instruction to start a series of processing for detecting the blood vessel is not received.

The user confirms the ultrasound image U sequentially generated by repeating step S11 and step S12, and for example, as illustrated in Fig. 2, in a case in which it is recognized that ultrasound image U including the blood vessel-like structures A1 to A3 of the subject to be examined is obtained, the instruction to start a series of processing for detecting the blood vessel is input via the input device 21. In such a manner, in a case in which the instruction to start a series of processing is input, in step S12, the apparatus control unit 20A determines that the instruction to start a series of processing is received, and proceeds to step S13.

In step S13, the ultrasound image U is generated in the similar manner as in step S11.

In step S14, the certainty calculation unit 13 analyzes the ultrasound image U generated in step S13 to calculate the certainties of the structures A1 to A3 in the ultrasound image U. The certainties of the blood vessels calculated for the structures A1 to A3 in such a manner are stored in the certainty memory 14 under the control of the apparatus control unit 20A.

In step S15, the apparatus control unit 20A determines whether or not the number of frames of the ultrasound image U for which the certainty of the blood vessel is calculated in step S14 is N. Here, N is an integer of 2 or more, and for example, N is set 20 to 100. At the moment, since the ultrasound image U for which the certainty of the blood vessel is calculated in step S14 has only one frame, it is determined that the number of frames of the ultrasound image U for which the certainty of the blood vessel is calculated in step S14 is not N and returns to step S13. In such a manner, step S13 to step S15 are repeated until the number of frames of the ultrasound image U for which the certainties of the blood vessels are calculated in step S14 becomes N, and the calculated certainties are sequentially stored in the certainty memory 14.

In step S15, in a case in which it is determined that the number of frames of the ultrasound image U for which the certainty of the blood vessel is calculated in step S14 is N, the processing proceeds to step S16.

In step S16, the certainty threshold value change unit 15 calculates the change value for the certainty threshold value of the blood vessel detection unit 16 based on the plurality of certainties calculated for the ultrasound image U having the N frame by repeating step S13 to step S15.

In step S17, the certainty threshold value change unit 15 changes an initial value of the certainty threshold value of the blood vessel detection unit 16 to the change value.

In step S18, the blood vessel detection unit 16 detects a blood vessel having a certainty higher than the certainty threshold value, which is changed in step S17, for the latest ultrasound image U, that is, the ultrasound image U generated at the end, in the repetition of the step S13 to step S15. For example, among the blood vessel-like structures A1 to A3 illustrated in Fig. 2, in a case in which the certainties of the structures A1 and A2 are higher than the certainty threshold value and the certainty of the structure A3 is lower than the certainty threshold value, the blood vessel detection unit 16 detects structures A1 and A2 as blood vessels.

Finally, in step S19, the blood vessel detection unit 16 highlights the structures A1 and A2 in the latest ultrasound image U detected as blood vessels in step S18 and displays the structures A1 and A2 on the monitor 12.

From the above, with the ultrasound diagnostic apparatus 1A according to the second embodiment of the present invention, since the certainty threshold value is changed based on the plurality of certainties calculated by the certainty calculation unit 13 for the ultrasound image U having N frame, even in a case in which the blood vessels in the ultrasound image U are difficult to see because of the state of the subject, the blood vessels can be detected with high accuracy in the same manner as the image processing apparatus 1 of the first embodiment.

As illustrated in Fig. 7, while the transmission and reception circuit 32 is comprised in the ultrasound probe 2, the transmission and reception circuit 32 may be comprised in the diagnostic apparatus main body 3 instead of being comprised in the ultrasound probe 2.

In addition, while the image generation unit 33 is comprised in the diagnostic apparatus main body 3, the image generation unit 33 may be comprised in the ultrasound probe 2 instead of being comprised in the diagnostic apparatus main body 3.

In addition, as illustrated in Fig. 9, while the image generation unit 33 comprises the signal processing unit 45, the DSC 46, and the image processing unit 47, the signal processing unit 45 can be included in the ultrasound probe 2.

In addition, a connection method between the ultrasound probe 2 and the diagnostic apparatus main body 3 is not particularly limited and may be wired connection or wireless connection.

In addition, the diagnostic apparatus main body 3 may be a so-called handheld type that can be easily carried by the user, or may be a so-called stationary type.

In addition, in step S12, in a case in which the instruction to start a series of processing for detecting the blood vessel is input by the user via the input device 21, the apparatus control unit 20A determines that the instruction has been received. However, the trigger for determining that the instruction to start a series of processing for detecting the blood vessel has been received is not particularly limited to the input of the instruction via the input device 21.

For example, the apparatus control unit 20A determines whether or not the ultrasound probe 2 is in contact with the body surface of the subject, in a case in which it is determined that the ultrasound probe 2 is in contact with the body surface of the subject, the apparatus control unit 20A also can determine that the instruction to start a series of processing for detecting the blood vessel has been received.

Regarding the determination of whether or not the ultrasound probe 2 is in contact with the body surface of the subject, for example, a pressure sensor (not illustrated) for measuring a contact pressure of the ultrasound probe 2 with respect to the body surface of the subject is attached to the ultrasound probe 2, and the apparatus control unit 20A can determine that the ultrasound probe 2 is in contact with the body surface of the subject in a case in which a pressure value measured by the pressure sensor exceeds a determined pressure threshold value, and can determine that the ultrasound probe 2 is away from the body surface of the subject in a case in which the measured pressure value is equal to or lower than the determined pressure threshold value.

In addition, in a case in which the ultrasound probe 2 is in contact with the body surface of the subject, an ultrasound image U having a certain brightness or higher corresponding to the tissue in the subject is captured. It is known that in a case in which the ultrasound probe 2 is away from the body surface of the subject, that is, in a so-called aerial radiation state, an ultrasound image U that is entirely filled with black because the ultrasound echo does not propagate to the transducer array 31 is captured. Therefore, the apparatus control unit 20A can determine that the ultrasound probe 2 is in contact with the body surface of the subject in a case in which the ultrasound image U having a certain brightness or higher is captured, and can determine that the ultrasound probe 2 is away from the body surface of the subject in a case in which the ultrasound image U that is entirely filled with black is captured.

In addition, for example, the apparatus control unit 20A can also determine that, in a case in which the capturing of the ultrasound image U is started, the instruction to start a series of processing for detecting the blood vessel has been received.

By these methods, by determining that the instruction to start a series of processing for detecting the blood vessel has been received, the user can save the trouble of performing the input operation via the input device 21 and can more smoothly execute a series of processing for detecting the blood vessel.

In addition, although the processing of step S13 to step S15 is repeated until the certainty of the blood vessel is calculated for the ultrasound image U having the determined N frame, the processing of step S13 to step S15 may be repeated until a determined time elapses from a time at which the processing of step S13 is first started. In this case, in step S15, the apparatus control unit 20A determines whether or not a determined time has elapsed since the start of the first step S13. The determined time can be set, for example, from 1 second to 5 seconds.

In addition, the determined number of frames of the ultrasound image U used for the determination of step S15 and an elapsed time from a time at which the first step S13 is started can be set in advance by the user via the input device 21.

In addition, the number of frames and the elapsed time can be set for each user who uses the ultrasound diagnostic apparatus 1A and can be held in the apparatus control unit 20A. In this case, for example, by authenticating the user who uses the ultrasound diagnostic apparatus 1A at the start of the examination of the subject, the number of frames or the elapsed time held corresponding to the authenticated user is read out, and the read number of frames or the read elapsed time can be used for the determination in step S 15.

Examples of the user authentication include, for example, a method of inputting an identifier corresponding to the user from the input device 21, a method of recognizing a user's fingerprint by providing a fingerprint sensor (not illustrated) in the ultrasound diagnostic apparatus 1A, a method of recognizing a user's face imaged by a camera (not illustrated) by providing the camera in the ultrasound diagnostic apparatus 1A, a method of recognizing a user's iris by imaging a user's eyes with a camera, a method of recognizing a voiceprint from a user's voice recorded by a microphone (not illustrated) by providing the microphone in the ultrasound diagnostic apparatus 1A, a method of reading a bar code (one-dimensional code) corresponding to the user, and a method of reading a two-dimensional code such as a quick response (QR) code (registered trademark) corresponding to the user.

In addition, in step S14, the apparatus control unit 20A stores the certainty in the certainty memory 14 each time the certainty is calculated by the certainty calculation unit 13, but the timing at which the certainty is stored is not particularly limited to this.

In general, in a case in which the ultrasound image U including the blood vessel is captured, the ultrasound probe 2 is often stationary. Therefore, for example, it is determined that the ultrasound probe 2 is stationary and in a case in which it is determined that the ultrasound probe 2 is stationary for a determined time or longer, the calculated certainty can be stored in the certainty memory 14.

For example, a motion sensor such as an acceleration sensor or a gyro sensor, a pressure sensor, or the like for detecting the motion of the ultrasound probe 2 is attached, and the apparatus control unit 20A can determine that the ultrasound probe 2 is stationary based on the values measured by those sensors.

In addition, in a case in which the ultrasound probe 2 is stationary, ultrasound images U similar to each other are often continuously captured. Therefore, the apparatus control unit 20A can, for example, calculate the similarity of the entire image between the ultrasound images U continuously generated by the image generation unit 33, and in a case in which the calculated similarity is equal to or larger than the determined similarity threshold value, the apparatus control unit 20A can determine that the ultrasound probe 2 is stationary.

In addition, since the certainty of the blood vessel is not calculated in a case in which the ultrasound probe 2 is away from the body surface of the subject, the apparatus control unit 20A can determine, for example, whether or not the ultrasound probe 2 is in contact with the body surface of the subject by analyzing the ultrasound image U or making a determination using the measured values of the pressure sensor attached to the ultrasound probe 2, can store the certainty in a case in which it is determined that the ultrasound probe 2 is in contact with the body surface of the subject, and can stop storing the certainty in a case in which it is determined that the ultrasound probe 2 is away from the body surface of the subject.

In addition, the moving speed of the ultrasound probe 2 is often relatively high in a case in which the user has not been able to image the blood vessel to be examined, and the moving speed of the ultrasound probe 2 is often relatively low in a case in which the user has been able to image the blood vessel to be examined. Therefore, the apparatus control unit 20A can determine whether or not the moving speed of the ultrasound probe 2 is lower than a determined moving speed, and can store the certainty in a case in which it is determined that the moving speed of the ultrasound probe 2 is lower than the determined moving speed.

For example, the moving speed of the ultrasound probe 2 can be measured by attaching an acceleration sensor (not illustrated) to the ultrasound probe 2. In this case, the apparatus control unit 20A can determine whether or not the moving speed measured by the sensor attached to the ultrasound probe 2 is lower than the determined moving speed.

In addition, it is considered that in a case in which the moving speed of the ultrasound probe 2 is low, the similarity between the ultrasound images U continuously generated by the image generation unit 33 is relatively large, and in a case in which the moving speed of the ultrasound probe 2 is high, the similarity between the continuously generated ultrasound images U is relatively small. Therefore, for example, the apparatus control unit 20A can calculate the similarity between frames continuously generated by the image generation unit 33, and can determine that the larger the calculated similarity, the higher the moving speed of the ultrasound probe 2, and the lower the calculated similarity, the lower the moving speed of the ultrasound probe 2. Therefore, the apparatus control unit 20A can estimate the moving speed of the ultrasound probe 2 from the similarity between the ultrasound images U continuously generated by the image generation unit 33, and can determine whether or not the estimated moving speed is lower than the determined moving speed.

In addition, the moving speed of the ultrasound probe 2 is often relatively high in a case in which the user has not been able to image the blood vessel to be examined, and the moving speed of the ultrasound probe 2 in a case in which the user has been able to image the blood vessel to be examined. Therefore, in order to improve the accuracy of the change value calculated by the certainty threshold value change unit 15, it is preferable to increase the number of stored certainties as the moving speed of the ultrasound probe 2 decreases, and to decrease the number of stored certainties as the moving speed of the ultrasound probe 2 increases.

Therefore, for example, the apparatus control unit 20A can store the certainty corresponding to the ultrasound image U selected at a longer frame interval as the moving speed of the ultrasound probe 2 increases, in the certainty memory 14, and can store the certainty corresponding to the ultrasound image U selected at a shorter frame interval as the moving speed of the ultrasound probe 2 decreases, in the certainty memory 14. As described above, the apparatus control unit 20A can store the certainty calculated based on the ultrasound image U having a frame selected by a frame interval according to the moving speed of the ultrasound probe 2 in the ultrasound image U having a plurality of frames generated by the image generation unit 33, in the certainty memory 14.

In addition, although the flowchart illustrated in Fig. 10 illustrates an aspect in which the certainty threshold value is changed only once, in a case in which the ultrasound images U are continuously generated even after the certainty threshold value is changed, the change value can be calculated each time the certainty is calculated for the ultrasound image U having the N frame, and the certainty threshold value of the blood vessel detection unit 16 can be changed to the change value. In this case, the certainty threshold value can be sequentially changed to the change value with higher accuracy.

In addition, in a case in which the ultrasound images U are continuously generated even after the certainty threshold value is changed, the certainty threshold value is changed a determined number of times, for example, once to five times, and thereafter, it is possible to stop changing the certainty threshold value. In a case in which the blood vessel in the ultrasound image U is not detected for some reason, it is difficult to specify whether the cause is due to the ultrasound image U or a change in the certainty threshold value. Therefore, by determining the number of times that the certainty threshold value can be changed, even in a case in which the blood vessel in the ultrasound image U is not detected, the user can easily determine the cause and can take appropriate measures.

In addition, the changed certainty threshold value can be reset to an initial value of the certainty threshold value. For example, the certainty threshold value change unit 15 can reset the value of the certainty threshold value by inputting a reset instruction by the input operation of the user via the input device 21. In addition, in a case in which the certainty threshold value is changed a plurality of times, the reset processing may be executed such that the certainty threshold value is changed to the past certainty threshold value by a determined number of changes, such as resetting the certainty threshold value to the certainty threshold value immediately before it is changed to the latest certainty threshold value instead of the initial value of the certainty threshold value. By resetting the certainty threshold value in such a manner, for example, even in a case in which the certainty threshold value deviates from an appropriate value for some reason, the certainty threshold value change unit 15 can change the certainty threshold value back to the appropriate value.

### Explanation of References

1: image processing apparatus
1A: ultrasound diagnostic apparatus
2: ultrasound probe
3: diagnostic apparatus main body
11: display control unit
12: monitor
13: certainty calculation unit
14: certainty memory
15: certainty threshold value change unit
16: blood vessel detection unit
17: certainty threshold value memory
18: manual change unit
19: change notification unit
20, 20A: apparatus control unit
21: input device
22, 22A: processor
31: transducer array
32: transmission and reception circuit
33: image generation unit
41: pulser
42: amplification unit
43: AD conversion unit
44: beam former
45: signal processing unit
46: DSC
47: image processing unit
A1 to A3: structure
B: seek bar
B1: slide button
C 1: execution button
C2: cancel button
P1, P2: dialog panel
U: ultrasound image

## Claims

1. An image processing apparatus (1, 1A) comprising:
a certainty calculation unit (13) configured to analyze an ultrasound image (U) of a subject for each frame and calculate a certainty of each blood vessel-like structure (A1, A2, A3) in the ultrasound image;
a blood vessel detection unit (16) configured to detect the blood vessel-like structures (A1, A2) for which the certainty calculated by the certainty calculation unit is higher than a certainty threshold value; and
a certainty threshold value change unit (15) configured to change the certainty threshold value based on a plurality of the certainties calculated for the ultrasound image having a plurality of frames by the certainty calculation unit.

2. The image processing apparatus (1A) according to claim 1, further comprising:
an ultrasound probe (2); and
an image generation unit (33) configured to generate the ultrasound image (U) analyzed by the certainty calculation unit (15) based on transmission and reception of an ultrasound beam using the ultrasound probe.

3. The image processing apparatus (1A) according to claim 2, further comprising:
a certainty memory (14) configured to store the certainty calculated by the certainty calculation unit (13).

4. The image processing apparatus (1A) according to claim 3, further comprising:
an apparatus control unit (20A) configured to control storage of the certainty in the certainty memory (14).

5. The image processing apparatus (1A) according to claim 4,
wherein the apparatus control unit (20A) is configured to:
determine whether the ultrasound probe (2) is stationary,
store the certainty in the certainty memory (14) in a case in which it is determined that the ultrasound probe is stationary for a determined time or longer, and
not store the certainty in the certainty memory in a case in which it is determined that the ultrasound probe is not stationary for the determined time or longer.

6. The image processing apparatus (1A) according to claim 4,
wherein the apparatus control unit (20A) is configured to:
determine whether the ultrasound probe (2) is in contact with a body surface of the subj ect,
store the certainty in the certainty memory (14) in a case in which it is determined that the ultrasound probe is in contact with the body surface of the subject, and
not store the certainty in the certainty memory in a case in which it is determined that the ultrasound probe is not in contact with the body surface of the subject.

7. The image processing apparatus (1A) according to claim 4,
wherein the apparatus control unit (20A) is configured to:
store the certainty in the certainty memory (14) in a case in which it is determined that a moving speed of the ultrasound probe is lower than a determined moving speed, and
not store the certainty in the certainty memory in a case in which it is determined that a moving speed of the ultrasound probe is equal to or higher than a determined moving speed.

8. The image processing apparatus (1A) according to claim 4,
wherein the apparatus control unit (20A) is configured to:
store the certainty in the certainty memory (14), the certainty being calculated based on the ultrasound image (U) having a frame selected by a frame interval according to a moving speed of the ultrasound probe (2) in the ultrasound image having a plurality of frames generated by the image generation unit (33).

9. The image processing apparatus (1, 1A) according to any one of claims 1 to 8,
wherein the certainty threshold value change unit (15) is configured to:
calculate a change value by multiplying a maximum value of the plurality of certainties calculated for the ultrasound image having a plurality of frames by a determined ratio, and
change the certainty threshold value to the change value.

10. The image processing apparatus (1, 1A) according to any one of claims 1 to 8,
wherein the certainty threshold value change unit (15) is configured to:
calculate a change value by statistically analyzing the plurality of certainties calculated for the ultrasound image having a plurality of frames, and
change the certainty threshold value to the change value.

11. The image processing apparatus (1, 1A) according to claim 9 or 10,
wherein the certainty threshold value change unit (15) is configured to:
change the certainty threshold value to the change value in a case in which the change value is lower than the certainty threshold value of the blood vessel detection unit (16), and
not change the certainty threshold value to the change value in a case in which the change value is equal to or higher than the certainty threshold value of the blood vessel detection unit.

12. The image processing apparatus (1, 1A) according to any one of claims 1 to 11, further comprising:
a change notification unit (19) configured to notify a user of a change of the certainty threshold value.

13. The image processing apparatus according (1, 1A) to any one of claims 1 to 12, further comprising:
a certainty threshold value memory (17) configured to store the certainty threshold value changed by the certainty threshold value change unit (15) for each subject.

14. The image processing apparatus (1, 1A) according to any one of claims 1 to 13, further comprising:
an input device (21) for a user to perform an input operation; and
a manual change unit (18) configured to change the certainty threshold value based on the input operation via the input device.

15. A control method of an image processing apparatus (1, 1A) comprising:
analyzing an ultrasound image (U) having a plurality of frames of a subject for each frame and calculating a certainty of each blood vessel-like structure in the ultrasound image; detecting the blood vessel-like structures in which the certainty is higher than a certainty threshold value; and changing the certainty threshold value based on a plurality of the certainties calculated for the ultrasound image having a plurality of frames.

## Patentansprüche

1. Bildverarbeitungsvorrichtung (1, 1A), umfassend:
eine Gewissheitsberechnungseinheit (13), die so konfiguriert ist, dass sie ein Ultraschallbild (U) einer Untersuchungsperson für jedes Einzelbild analysiert und eine Gewissheit jeder blutgefäßähnlichen Struktur (A1, A2, A3) in dem Ultraschallbild berechnet;
eine Blutgefäß-Detektionseinheit (16), die so konfiguriert ist, dass sie die blutgefäßähnlichen Strukturen (A1, A2), für die die von der Gewissheitsberechnungseinheit berechnete Gewissheit höher als ein Gewissheitsschwellenwert ist, detektiert; und
eine Gewissheitsschwellenwert-Änderungseinheit (15), die so konfiguriert ist, dass sie den Gewissheitsschwellenwert auf der Grundlage mehrerer der Gewissheiten ändert, die von der Gewissheitsberechnungseinheit für das Ultraschallbild, das mehrere Einzelbilder aufweist, berechnet wurden.

2. Bildverarbeitungsvorrichtung (1A) nach Anspruch 1, ferner umfassend:
eine Ultraschallsonde (2); und
eine Bilderzeugungseinheit (33), die so konfiguriert ist, dass sie das Ultraschallbild (U), das von der Gewissheitsberechnungseinheit (15) analysiert wird, auf der Grundlage von Übertragung und Empfang eines Ultraschallstrahls unter Verwendung der Ultraschallsonde erzeugt.

3. Bildverarbeitungsvorrichtung (1A) nach Anspruch 2, ferner umfassend:
einen Gewissheitsspeicher (14), der so konfiguriert ist, dass er die von der Gewissheitsberechnungseinheit (13) berechnete Gewissheit speichert.

4. Bildverarbeitungsvorrichtung (1A) nach Anspruch 3, ferner umfassend:
eine Vorrichtungssteuereinheit (20A), die so konfiguriert ist, dass sie Speicherung der Gewissheit im dem Gewissheitsspeicher (14) steuert.

5. Bildverarbeitungsvorrichtung (1A) nach Anspruch 4,
wobei die Vorrichtungssteuereinheit (20A) so konfiguriert ist, dass sie:
bestimmt, ob die Ultraschallsonde (2) stationär ist,
die Gewissheit im dem Gewissheitsspeicher (14) in einem Fall, in dem bestimmt wird, dass die Ultraschallsonde für eine bestimmte Zeit oder länger stationär ist, speichert, und
nicht die Gewissheit im dem Gewissheitsspeicher in einem Fall, in dem bestimmt wird, dass die Ultraschallsonde nicht für die bestimmte Zeit oder länger stationär ist, speichert.

6. Bildverarbeitungsvorrichtung (1A) nach Anspruch 4,
wobei die Vorrichtungssteuereinheit (20A) so konfiguriert ist, dass sie:
bestimmt, ob die Ultraschallsonde (2) mit einer Körperoberfläche der Untersuchungsperson in Kontakt steht oder nicht,
die Gewissheit in dem Gewissheitsspeicher (14) in einem Fall, in dem bestimmt wird, dass die Ultraschallsonde mit der Körperoberfläche der Untersuchungsperson in Kontakt steht, speichert, und
nicht die Gewissheit in dem Gewissheitsspeicher in einem Fall, in dem bestimmt wird, dass die Ultraschallsonde nicht mit der Körperoberfläche der Untersuchungsperson in Kontakt steht, speichert.

7. Bildverarbeitungsvorrichtung (1A) nach Anspruch 4,
wobei die Vorrichtungssteuereinheit (20A) so konfiguriert ist, dass sie:
die Gewissheit in dem Gewissheitsspeicher (14) in einem Fall, in dem bestimmt wird, dass eine Bewegungsgeschwindigkeit der Ultraschallsonde niedriger als eine bestimmte Bewegungsgeschwindigkeit ist, speichert, und
nicht die Gewissheit in dem Gewissheitsspeicher in einem Fall, in dem bestimmt wird, dass eine Bewegungsgeschwindigkeit der Ultraschallsonde gleich oder höher als eine bestimmte Bewegungsgeschwindigkeit ist, speichert.

8. Bildverarbeitungsvorrichtung (1A) nach Anspruch 4,
wobei die Vorrichtungssteuereinheit (20A) so konfiguriert ist, dass sie:
die Gewissheit in dem Gewissheitsspeicher (14) speichert, wobei die Gewissheit auf der Grundlage des Ultraschallbildes (U), das ein Einzelbild aufweist, das durch ein Einzelbildintervall gemäß einer Bewegungsgeschwindigkeit der Ultraschallsonde (2) ausgewählt wird, in dem Ultraschallbild, das mehrere Einzelbilder aufweist, die von der Bilderzeugungseinheit (33) erzeugt wurden, berechnet wird.

9. Bildverarbeitungsvorrichtung (1, 1A) nach einem der Ansprüche 1 bis 8,
wobei die Gewissheitsschwellenwert-Änderungseinheit (15) so konfiguriert ist, dass sie:
einen Änderungswert durch Multiplizieren eines Maximalwerts der mehreren Gewissheiten, die für das Ultraschallbild, das mehrere Einzelbilder aufweist, berechnet wurden, mit einem bestimmten Verhältnis berechnet, und
den Gewissheitsschwellenwert auf den Änderungswert ändert.

10. Bildverarbeitungsvorrichtung (1, 1A) nach einem der Ansprüche 1 bis 8,
wobei die Gewissheitsschwellenwert-Änderungseinheit (15) so konfiguriert ist, dass sie:
einen Änderungswert durch statistisches Analysieren der mehreren Gewissheiten, die für das Ultraschallbild, das mehrere Einzelbilder aufweist, berechnet wurden, berechnet, und
den Gewissheitsschwellenwert auf den Änderungswert ändert.

11. Bildverarbeitungsvorrichtung (1, 1A) nach Anspruch 9 oder 10,
wobei die Gewissheitsschwellenwert-Änderungseinheit (15) so konfiguriert ist, dass sie:
den Gewissheitsschwellenwert auf den Änderungswert in einem Fall, in dem der Änderungswert niedriger als der Gewissheitsschwellenwert der Blutgefäß-Detektionseinheit (16) ist, ändert, und
nicht den Gewissheitsschwellenwert auf den Änderungswert in einem Fall, in dem der Änderungswert gleich oder höher als der Gewissheitsschwellenwert der Blutgefäß-Detektionseinheit ist, ändert.

12. Bildverarbeitungsvorrichtung (1, 1A) nach einem der Ansprüche 1 bis 11, ferner umfassend:
eine Änderungsbenachrichtigungseinheit (19), die so konfiguriert ist, dass sie einen Benutzer über eine Änderung des Gewissheitsschwellenwerts benachrichtigt.

13. Bildverarbeitungsvorrichtung nach (1, 1A) einem der Ansprüche 1 bis 12, ferner umfassend:
einen Gewissheitsschwellenwert-Speicher (17), der so konfiguriert ist, dass er den Gewissheitsschwellenwert, der von der Gewissheitsschwellenwert-Änderungseinheit (15) geändert wird, für jede Untersuchungsperson speichert.

14. Bildverarbeitungsvorrichtung (1, 1A) nach einem der Ansprüche 1 bis 13, ferner umfassend:
eine Eingabevorrichtung (21), durch die ein Benutzer eine Eingabebedienung durchführt; und
eine manuelle Änderungseinheit (18), die so konfiguriert ist, dass sie den Gewissheitsschwellenwert auf der Grundlage der Eingabebedienung via die Eingabevorrichtung ändert.

15. Steuerverfahren einer Bildverarbeitungsvorrichtung (1, 1A), umfassend:
Analysieren eines Ultraschallbildes (U), das mehrere Einzelbilder einer Untersuchungsperson aufweist, für jedes Einzelbild und Berechnen einer Gewissheit jeder blutgefäßähnlichen Struktur in dem Ultraschallbild;
Detektieren der blutgefäßähnlichen Strukturen, wobei die Gewissheit höher als ein Gewissheitsschwellenwert ist; und
Ändern des Gewissheitsschwellenwerts auf der Grundlage mehrerer der Gewissheiten, die für das Ultraschallbild, das mehrere Einzelbilder aufweist, berechnet wurden.

## Revendications

1. Appareil de traitement d'images (1, 1A) comprenant :
une unité de calcul de certitude (13) configurée pour analyser une image ultrasonore (U) d'un sujet pour chaque trame et calculer une certitude de chaque structure semblable à un vaisseau sanguin (A1, A2, A3) dans l'image ultrasonore ;
une unité de détection des vaisseaux sanguins (16) configurée pour détecter les structures semblables à des vaisseaux sanguins (A1, A2) pour lesquelles la certitude calculée par l'unité de calcul de certitude est supérieure à une valeur seuil de certitude ; et
une unité de changement de valeur seuil de certitude (15) configurée pour changer la valeur seuil de certitude sur la base d'une pluralité de certitudes calculées pour l'image ultrasonore comportant une pluralité de trames par l'unité de calcul de certitude.

2. Appareil de traitement d'images (1A) selon la revendication 1, comprenant en outre :
une sonde ultrasonore (2) ; et
une unité de génération d'images (33) configurée pour générer l'image ultrasonore (U) analysée par l'unité de calcul de certitude (15) sur la base de la transmission et de la réception d'un faisceau ultrasonore à l'aide de la sonde ultrasonore.

3. Appareil de traitement d'images (1A) selon la revendication 2, comprenant en outre :
une mémoire de certitude (14) configurée pour stocker la certitude calculée par l'unité de calcul de certitude (13).

4. Appareil de traitement d'images (1A) selon la revendication 3, comprenant en outre :
une unité de contrôle d'appareil (20A) configurée pour contrôler le stockage de la certitude dans la mémoire de certitude (14).

5. Appareil de traitement d'images (1A) selon la revendication 4,
dans lequel l'unité de contrôle de l'appareil (20A) est configurée pour :
déterminer si la sonde ultrasonore (2) est stationnaire,
stocker la certitude dans la mémoire de certitude (14) dans un cas où il est déterminé que la sonde ultrasonore est stationnaire pendant une durée déterminé ou plus longtemps, et
ne pas stocker la certitude dans la mémoire de certitude dans un cas où il est déterminé que la sonde ultrasonore n'est pas stationnaire pendant la durée déterminé ou plus longtemps.

6. Appareil de traitement d'images (1A) selon la revendication 4,
dans lequel l'unité de contrôle de l'appareil (20A) est configurée pour :
déterminer si la sonde ultrasonore (2) est en contact avec une surface corporelle du sujet,
stocker la certitude dans la mémoire de certitude (14) dans un cas où il est déterminé que la sonde ultrasonore est en contact avec la surface corporelle du sujet, et
ne pas stocker la certitude dans la mémoire de certitude dans un cas où il est déterminé que la sonde ultrasonore n'est pas en contact avec la surface corporelle du sujet.

7. Appareil de traitement d'images (1A) selon la revendication 4,
dans lequel l'unité de contrôle de l'appareil (20A) est configurée pour :
stocker la certitude dans la mémoire de certitude (14) dans un cas où il est déterminé que une vitesse de déplacement de la sonde ultrasonore est inférieure à une vitesse de déplacement déterminée, et
ne pas stocker la certitude dans la mémoire de certitude dans un cas où il est déterminé que une vitesse de déplacement de la sonde ultrasonore est égale ou supérieure à une vitesse de déplacement déterminée.

8. Appareil de traitement d'images (1A) selon la revendication 4,
dans lequel l'unité de contrôle de l'appareil (20A) est configurée pour :
stocker la certitude dans la mémoire de certitude (14), la certitude étant calculée sur la base de l'image ultrasonore (U) comportant une trame sélectionnée par un intervalle de trame en fonction d'une vitesse de déplacement de la sonde ultrasonore (2) dans l'image ultrasonore comportant une pluralité de trames générées par l'unité de génération d'images (33).

9. Appareil de traitement d'images (1, 1A) selon l'une quelconque des revendications 1 à 8,
dans lequel l'unité de changement de valeur seuil de certitude (15) est configurée pour :
calculer une valeur de changement en multipliant une valeur maximale de la pluralité de certitudes calculées pour l'image ultrasonore comportant une pluralité de trames par un rapport déterminé, et
changer la valeur seuil de certitude à la valeur de changement.

10. Appareil de traitement d'images (1, 1A) selon l'une quelconque des revendications 1 à 8,
dans lequel l'unité de changement de valeur seuil de certitude (15) est configurée pour :
calculer une valeur de changement en analysant statistiquement la pluralité de certitudes calculées pour l'image ultrasonore comportant une pluralité de trames, et changer la valeur seuil de certitude à la valeur de changement.

11. Appareil de traitement d'images (1, 1A) selon la revendication 9 ou la revendication 10,
dans lequel l'unité de changement de valeur seuil de certitude (15) est configurée pour :
changer la valeur seuil de certitude à la valeur de changement dans un cas où la valeur de changement est inférieure à la valeur seuil de certitude de l'unité de détection des vaisseaux sanguins (16), et
ne pas changer la valeur seuil de certitude à la valeur de changement dans un cas où la valeur de changement est égale ou supérieure à la valeur seuil de certitude de l'unité de détection des vaisseaux sanguins.

12. Appareil de traitement d'images (1, 1A) selon l'une quelconque des revendications 1 à 11, comprenant en outre :
une unité de notification de changement (19) configurée pour notifier un utilisateur d'un changement de la valeur seuil de certitude.

13. Appareil de traitement d'images selon (1, 1A) l'une quelconque des revendications 1 à 12, comprenant en outre :
une mémoire de valeur seuil de certitude (17) configurée pour stocker la valeur seuil de certitude changée par l'unité de changement de valeur seuil de certitude (15) pour chaque sujet.

14. Appareil de traitement d'images (1, 1A) selon l'une quelconque des revendications 1 à 13, comprenant en outre :
dispositif d'entrée (21) permettant à un utilisateur d'effectuer une opération d'entrée ; et
une unité de changement manuelle (18) configurée pour changer la valeur seuil de certitude sur la base de l'opération d'entrée via le dispositif d'entrée.

15. Procédé de contrôle d'un appareil de traitement d'images (1, 1A) comprenant :
analyser une image ultrasonore (U) comportant une pluralité de trames d'un sujet pour chaque trame et calculer une certitude de chaque structure semblable à un vaisseau sanguin dans l'image ultrasonore ;
détecter les structures semblables à des vaisseaux sanguins dans lesquelles la certitude est supérieure à une valeur seuil de certitude ; et
changer la valeur seuil de certitude sur la base d'une pluralité des certitudes calculées pour l'image ultrasonore comportant une pluralité de trames.
